Europäisches Patentamt

(19) European Patent Office

Office européen des brevets

(11) EP 0 875 580 A1

(12) **EUROPEAN PATENT APPLICATION**

(43) Date of publication:
04.11.1998 Bulletin 1998/45

(51) Int. Cl.$^6$: **C12P 19/04**, A21D 2/18

(21) Application number: 97200981.5

(22) Date of filing: 02.05.1997

(84) Designated Contracting States:
AT BE CH DE DK ES FI FR GB GR IE IT LI LU NL PT SE

(71) Applicant: **Difass S.A.**
**47031 Borgo Maggiore (SM)**

(72) Inventors:
• **Puglisi, Pierpaolo**
**43100 Parma (IT)**

• **Ferrero, Iliana**
**43100 Parma (IT)**

(74) Representative:
**Ferreccio, Rinaldo et al**
**Jacobacci & Perani S.p.A.,**
**Via Visconti di Modrone 7**
**20122 Milano (IT)**

(54) **Process for obtaining polysaccharides from yeasts**

(57) A process for obtaining polysaccharides, in particular glucans and mannans, from alcoholic fermentation yeasts or lactose-fermenting yeasts, this process comprising the stages of:

- preparing a suspension of the said yeasts in an aqueous solution at a pH of between 2.0 and 5.0, at room temperature, with a solids content of 8%-20% w/w,
- subjecting the said suspension to homogenization at a temperature of 80-100°C, and
- separating the solid component from the suspension by centrifugation, this component consisting essentially of said polysaccharides.

The polysaccharides obtained using the present process are useful as additives for the food industry, in particular in the preparation of bread and similar baked products.

EP 0 875 580 A1

## Description

The present invention generally relates to the technical field of polysaccharides used in the food industry and the field of dietary products.

In particular, the invention relates to a process for obtaining polysaccharides from alcoholic fermentation yeasts or lactose-fermenting yeasts.

More specifically, the invention relates to a process for obtaining glucans and mannans from the cell walls of alcoholic fermentation yeasts or lactose-fermenting yeasts.

Polysaccharides from the glucan and mannan class are notoriously indigestible and therefore do not constitute a source of simple carbohydrates for human nutrition; nevertheless, they are highly useful molecules in the dietary and food industry, on account of some of their particular physicochemical properties.

The most important of these properties is that of absorbing and retaining large amounts of aqueous liquids. It is on account of this property that polysaccharides obtained from natural sources such as, for example, guar, carrageenan, xanthans and the like are used as dietary supplements for the purpose of improving gastrointestinal function.

The polysaccharides mentioned above are moreover widely used in the food sector for their thickening and stabilizing properties, usually in amounts of about 0.1-1.0%. Examples of foods to which polysaccharides are added are creamy cheeses, sauces, icecreams, cured pork products, jellies, cream desserts, etc.

In addition, their use as additives in biscuits and similar baked products entails a range of advantages associated, on the one hand, with the beneficial effect that foods thus prepared have on gastrointestinal function and, on the other hand, with the improved conservation properties of this food.

This is because, owing to the bioabsorbent effect that the abovementioned polysaccharides have on water, the latter is held for a long time and is simultaneously made unavailable for the massive growth of micro-organisms and the growth of small microbial populations which would impair the organoleptic properties of the product.

The prolonged retention of water within baked products considerably slows down the process by which they turn stale, thereby extending their "shelf-life".

The fact that the water is unavailable, evidenced by the low Aw value of the baked products to which the abovementioned polysaccharides are added, prevents, or in any case hinders, the growth of bacteria and other micro-organisms, thus preserving the product's characteristics of micro-biological purity, as well as its organoleptic properties, over a long period of time.

However, the polysaccharides referred to hitherto constitute ingredients which are foreign to the traditional composition accepted by current legislation governing bread products, this composition essentially comprising flour, water, bakers' yeast and optionally salt and fats or natural oils.

The problem therefore exists of providing natural polysaccharides which are endowed with the desired bio-adsorbent properties, which are derived from sources that, from a nutritional and health view point, are extremely safe and which are fairly inexpensive.

The way in which it was thought to solve the abovementioned problem was to use, as sources of the abovementioned polysaccharides, yeasts which have a proven historical tradition in foods, such as alcoholic fermentation yeasts, and in particular brewers' yeast, and lactose-fermenting yeasts.

These yeasts have a cell wall which represents 15-20% of the dry weight of the cell and which is made up of 80-90% by weight of glucans and mannans. The remaining components of the cell wall are represented by amino acids, enzymatic proteins and other polypeptides and lipids.

In accordance with the abovementioned solution, the problem underlying the present invention is solved by providing a process for obtaining polysaccharides of the class comprising glucans and mannans from alcoholic fermentation yeasts or lactose-fermenting yeasts, said process comprising the stages of preparing a suspension of the said yeasts in an aqueous solution at a pH of between 2.0 and 5.0, at room temperature, with a solids content of yeasts of 8%-20% w/w, subjecting the said suspension to homogenization at a temperature of 80-100°C and separating the solid component from the suspension by centrifugation, this component consisting essentially of said polysaccharides.

The abovementioned homogenization is preferably carried out at a pressure of 500-800 bar, advantageously at 600 bar.

According to one embodiment of the invention, the homogenization at a temperature of 80-100°C is carried out by preheating the suspension (to about 70-90°C) and then subjecting it to homogenization.

According to an alternative embodiment, the homogenization at 80-100°C is carried out by subjecting the initial suspension at room temperature to repeated stages of homogenization, until the abovementioned temperature of 80-100°C is reached as a consequence of the heat generated as a result of the intense mechanical treatment to which the suspension is subjected.

The pH of the suspension is preferably between 4 and 4.5 and is advantageously determined by the presence of a weak acid in the suspension.

Weak acids which are particularly suitable for the purposes of the process according to the invention are chosen from the group comprising acetic acid, trichloroacetic acid, lactic acid and citric acid.

Among the yeasts which may be used in the process according to the invention, those chosen from the group comprising Saccharomyces cerevisiae, Saccharomyces Carlsbergensis, Kluyveromyces marxianus and synonyms thereof, such as Kluyveromyces lactis and Kluyveromyces fragilis, are preferred.

Saccharomyces cerevisiae is particularly preferred.

The product obtained using the process according to the invention essentially consists, as has already been stated, of polysaccharides and may contain traces of fully-denatured polypeptide components which are therefore not available as subfermenting sources of carbon.

If it is desired to obtain polysaccharides consisting essentially of mannans, it is possible to follow the process described above by a subsequent stage consisting in treating the said solid component with a β-1,3-glucan hydrolase or with a natural extract containing it.

A natural extract which is useful for the above-mentioned purpose is malt must, or wort.

Treatment of the abovementioned solid component with β-1,3-glucan hydrolase or with wort is carried out by suspending the solid component in an aqueous solution containing 15-50 U/ml of β-1,3-glucan hydrolase or wort in an amount such as to give the same concentration of β-1,3-glucan hydrolase, at a pH of 4-6 and at a temperature of 25-35°C, for 15 minutes.

At the end of said treatment, further centrifugation is carried out in order to separate out a solid component consisting essentially of mannans.

By means of the process according to the invention, a product is obtained which essentially consists of polysaccharides, except for traces of polypeptides which are, in any case, devoid of any enzymatic activity since they have been completely denatured during the heat-treatment of the process and are therefore not capable of degrading the polysaccharides at all.

The other components of the cell wall of the yeasts, in other words the amino acids and lipids are not present in the final product of the process according to the invention, not even in trace amounts, since they have been completely dissolved or, at any rate, transferred into the liquid component separated out at the end of the centrifugation.

The polysaccharides obtained using the process according to the present invention have a moisture content of about 30%. Depending on the particular production or distribution requirements, the abovementioned polysaccharides may optionally be dried using any method conventionally employed in the food industry, and then stored away from contact with atmospheric moisture (hermetically sealed packaging).

The polysaccharides obtained according to the process of the present invention have a range of advantages over the polysaccharides currently used in the dietary and food industry.

Firstly, these polysaccharides have the capacity of adsorbing up to 200 times their own weight in water; moreover, they are derived from a highly reliable natural source, since alcoholic fermentation yeasts and lactose-fermenting yeasts have been used for centuries in the preparation of food and drink and are entirely safe from the hygiene/health point of view.

Consequently, the use of these polysaccharides for dietary purposes and their addition to foods in order to exploit their food-stabilizing properties must likewise be considered safe, not least because the process used for their extraction from yeasts does not involve the use of toxic or harmful substances.

Moreover, the cost of the polysaccharides obtained by the process according to the invention is decidedly low, by virtue of the fact that it is possible to use yeasts which are left over from food processing. For example, one source of yeasts which are suitable for the purposes of the present process consists of the biomass of brewers' yeast which settles out in beer fermentation vats at the end of the fermentation.

The biomass of brewers' yeast mentioned above constitutes a low-cost material since it is currently used only as an ingredient in livestock feeds or as a starting material for obtaining yeast extract.

It should also be pointed out that the liquid component that separates out at the end of the stage of centrifugation of the suspension subjected to homogenization according to the present process constitutes an advantageous by-product which can be used as a starting material in preparing a yeast extract having an amino acid content to simple carbohydrate content ratio which is greater than that of traditional yeast extracts obtained by alkaline hydrolysis, with obvious advantages from the nutritional point of view.

It should moreover be noted that the process according to the invention does not involve the production of any waste which then has to be carried away for disposal, since both of the components separated out at the end of the process can be used in the livestock or food sector.

From the above, it is clear that the present process is characterized by an appreciable operational saving and by a low, if not zero, environmental impact.

Lastly, it should be recalled that among the many possible applications presented by the polysaccharides obtained using the process according to the invention, which have been given as examples above with reference to the polysaccharides of the prior art, a particularly advantageous application is in the field of baked products and in particular bread.

It is known that so-called "white" bread has organoleptic properties which are generally preferred to those of bread prepared from wholemeal flour or by adding a certain amount of dietary fibre or bran.

It is also known that white bread tends to go stale much more quickly than wholemeal bread, precisely because it contains no bran and so cannot take advantage of the water-retaining effects of bran and the consequent delaying effect on the bread-hardening process.

The addition of a suitable amount of the polysaccharides obtained by the process according to the invention to the dough from which white bread is prepared allows the same delaying effect (and in fact, an even better effect) to be obtained as that provided by bran, without having any negative impact on the organoleptic properties of the final white bread.

The absence of any detrimental effects on the organoleptic properties of the bread (or of any other foodstuff) is ensured by the high-temperature treatment envisaged by the present process, which allows the removal of all the low-boiling components liable to impart particular unpleasant flavours or odours to the final polysaccharides.

Everything which has been outlined above is achieved by adding an additive which is not only of entirely natural origin but is actually obtained from one of the standard ingredients of bread, namely brewers' yeast.

The polysaccharides obtained by the present method may be added to traditional bread doughs or to similar baked products, in amounts such as to constitute 0.5-2.0% of the weight of the baked product.

Further advantages of the process according to the invention and of the products obtained thereby will become apparent from the examples given below by way of non-limiting illustration.

EXAMPLE 1

Brewers' yeast (Saccharomyces cerevisiae) obtained from the fermentation of a must for the production of beer was used as starting material, supplied in the form of a compressed biomass with a solids content of about 20% w/w.

10 kg of the abovementioned biomass were suspended in 10 litres of 20% w/v acetic acid solution at room temperature.

The suspension thus obtained was heated at a temperature of 80°C for 10 minutes and then fed into a Bartoli M21/B1 model single-stage homogenizer at a rate of 2.5 litres per minute.

The homogenizer was adjusted to work at a pressure of 700 bar.

The temperature of the suspension leaving the homogenizer was about 94°C.

At the end of the homogenization stage, the vital titre was checked in order to confirm that the yeast cells had ruptured.

The vital titre was checked either by the methylene blue method or by dilution and subsequent plating out.

In accordance with the first method, 5 ml of the homogenized suspension were diluted with water to a volume of 1 litre, with stirring.

5 ml of the suspension thus diluted were mixed with 5 ml of an aqueous methylene blue solution buffered to a pH of about 4.6, which was in turn prepared by mixing a 0.02% weight/volume solution of methylene blue in water with an equal volume of a solution obtained by mixing 99.75 ml of aqueous 2.72% w/v $KH_2PO_4$ solution with 0.25 ml of an aqueous 3.56% w/v $Na_2HPO_4$ solution.

After leaving the diluted sample with the methylene blue solution to incubate at 35°C for 10 minutes, the stained cells and the total number of cells were counted using a Bürker haemocytometer and microscope or a Thoma counting chamber.

The percentage of dead cells were calculated according to the following formula:

$$\frac{\text{number of stained cells} \times 100}{\text{number of stained cells} + \text{number of unstained cells}}$$

Both methods demonstrated that the number of living cells at the end of the homogenization was less than 1%.

The homogenized suspension was then subjected to centrifugation at 1500 × g at room temperature for 10 minutes in order to separate the solid component from the liquid component.

The solid component collected at the end of the centrifugation was in the form of a white powder consisting essentially of polysaccharides, in particular glucans and mannans, weighed 590 g and had a solids content of 70%.

EXAMPLE 2

10 kg of the same biomass of brewers' yeast used in the above example were suspended in 10 litres of 20% w/v trichloroacetic acid solution at room temperature.

The suspension thus obtained was fed into the same homogenizer used in Example 1, at a rate of 2.5 litres per minute.

The homogenizer was adjusted to work at a pressure of 600 bar.

The temperature of the suspension leaving the homogenizer was about 35°C.

The suspension was then subjected to a second and third passage through the homogenizer at the same pressure and the same feed rate.

After the second and third passages, the temperature of the suspension was equal to 63°C and 93°C respectively.

A determination of the vital titre according to the methods given in Example 1, carried out after each passage through the homogenizer, gave values of 92% (after the first passage), 37% (after the second passage) and <1% (after the third passage).

The suspension obtained after the third passage through the homogenizer was then subjected to centrifugation at 1500 × g at room temperature for 10 minutes in order to separate the solid component from the liquid component.

The solid component collected after centrifuging (white powder) consisted essentially of polysaccharides, in particular glucans and mannans, weighed 610 g and had a solids content of 70%.

EXAMPLE 3

500 g of the solid component collected after the centrifugation in Example 1 were suspended in 4 litres of a malt must used in the production of beer, acidified to pH 4.5 with acetic acid and containing $\beta$-1,3-glucan hydrolase in concentrations equal to about 40 U/ml, at a temperature of 35°C.

The suspension was stirred for 4 hours, after which it was centrifuged at 1500 × g for 10 minutes at room temperature.

The solid component separated out by centrifugation (150 g of white powder with a solids content of 70%) consisted essentially of mannans.

EXAMPLE 4

An amount of polysaccharides, prepared according to Example 1, equal to 1% of the total weight of the dough was added to a bread dough comprising 00 type wheat flour, water, salt and bakers' yeast in the usual proportions.

The dough was then divided up and shaped so as to obtain, after baking at about 200°C, bread rolls weighing about 70-80 g.

The rolls thus obtained were subjected to a storage test at room temperature, compared with rolls prepared from a dough having the same composition but not supplemented with the polysaccharides according to the invention.

The time it took for the rolls containing the polysaccharides according to the invention and the control rolls to go stale was evaluated by means of an organoleptic test carried out by tasting panels.

The rolls containing the polysaccharides according to the invention took about 5 times as long to go stale as the control rolls.

**Claims**

1. A process for obtaining polysaccharides, in particular glucans and mannans, from alcoholic fermentation yeasts or lactose-fermenting yeasts, the said process comprising the stages of:

   - preparing a suspension of the said yeasts in an aqueous solution at a pH of between 2.0 and 5.0, at room temperature, with a solids content of 8%-20% w/w,
   - subjecting the said suspension to homogenization at a temperature of 80-100°C, and
   - separating the solid component from the suspension by centrifugation, this component consisting essentially of said polysaccharides.

2. A process according to Claim 1, characterized in that said homogenization is carried out at a pressure of 500-800 bar.

3. A process according to Claim 1, characterized in that said homogenization is carried out at a pressure of 600 bar.

4. A process according to any one of the preceding claims, characterized in that said homogenization at a temperature of 80-100°C is carried out by preheating the suspension and then subjecting it to homogenization.

5. A process according to any one of Claims 1 to 3, characterized in that said homogenization at 80-100°C is carried out by subjecting the suspension prepared at room temperature to repeated stages of homogenization until the said temperature of 80-100°C is reached as a consequence of the heat generated as a result of the intense mechanical treatment to which the suspension is subjected.

6.  A process according to Claim 5, characterized in that said suspension prepared at room temperature is subjected to three successive stages of homogenization at 600 bar.

7.  A process according to any one of the preceding claims, characterized in that the pH of the suspension is preferably between 4 and 4.5.

8.  A process according to Claim 7, characterized in that said suspension comprises a weak acid chosen from the group comprising acetic acid, trichloroacetic acid, tartaric acid, glycolic acid and citric acid.

9.  A process according to any one of the preceding claims, characterized in that said yeasts are chosen from the group comprising Saccharomyces cerevisiae, Saccharomyces carlsbergensis, Kluyveromyces marxianus and synonyms thereof, such as Kluyveromyces lactis and Kluyveromyces fragilis.

10. A process according to Claim 9, characterized in that said yeasts belong to the species Saccharomyces cerevisiae.

11. A process according to any one of the preceding claims, characterized in that it further comprises a stage consisting in treating said solid component with a β-1,3-glucan hydrolase or with a natural extract containing it.

12. A process according to Claim 11, characterized in that said natural extract is malt must.

13. A process according to Claims 11 or 12, characterized in that the treatment of said solid component with β-1,3-glucan hydrolase or with malt must is carried out by suspending the solid component in an aqueous solution containing 15-50 U/ml of β-1,3-glucan hydrolase or malt must in an amount such as to give the same concentration of β-1,3-glucan hydrolase, at a pH of 4-6 and at a temperature of 25-35°C, for 2-5 hours.

14. A process according to any one of Claims 11 to 13, characterized in that it comprises a centrifugation stage at the end of said treatment, in order to separate out a solid component consisting essentially of mannans.

15. Polysaccharides obtainable by a process according to any one of the preceding claims.

16. Polysaccharides obtainable by the process according to Claim 10.

17. Use of the polysaccharides according to either of Claims 15 and 16 as thickeners and/or stabilizers in food.

18. Use of the polysaccharides according to either of Claims 15 and 16 as additives in doughs for bread and similar baked products in order to obtain a longer shelf-life of the resulting baked products.

19. Bread and similar baked products, characterized in that they contain from 0.5 to 2.0% by weight of the polysaccharides according to either of Claims 15 and 16.

**European Patent Office**

**EUROPEAN SEARCH REPORT**

Application Number

EP 97 20 0981

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | US 5 082 936 A (JAMAS ET AL.) 21 January 1992 <br> * column 6, line 60 - column 7, line 25 * | 1-19 | C12P19/04 <br> A21D2/18 |
| Y | US 3 867 554 A (SUCHER ET AL.) 18 February 1975 <br> * column 6, line 22 - line 38 * | 1-19 | |
| X | US 5 250 436 A (JAMAS ET AL.) <br> * table 7 * | 15-17 | |
| X | US 4 962 094 A (JAMAS ET AL.) <br> * column 5, line 10 - column 6, line 65 * | 15-17 | |
| A | WO 91 07091 A (DONZIS B.) 30 May 1991 | | |
| A | EP 0 416 343 A (CONSIGLIO NAZIONALE DELLE RICHERCHE) 13 March 1991 | | |

| | TECHNICAL FIELDS SEARCHED (Int.Cl.6) |
|---|---|
| | C12P <br> C08B <br> A23D <br> A21D |

The present search report has been drawn up for all claims

| Place of search | Date of completion of the search | Examiner |
|---|---|---|
| THE HAGUE | 15 April 1998 | Lensen, H |